# EUROPEAN PATENT SPECIFICATION

(11) **EP 2 379 741 B1**
(45) Date of publication and mention of the grant of the patent: **23.03.2016**
(21) Application number: 09795713.8
(22) Date of filing: 17.12.2009
(51) Int. Cl.: C12Q 1/68

(54) **METHOD FOR DETECTING A TARGET NUCLEIC ACID IN A SAMPLE**
VERFAHREN ZUM NACHWEISEN EINER ZIELNUKLEINSÄURE IN EINER PROBE
MÉTHODE DE DÉTECTION D'UN ACIDE NUCLÉIQUE CIBLE DANS UN ÉCHANTILLON

(30) Priority: 22.12.2008 US 341130
(43) Date of publication of application: 26.10.2011
(73) Proprietor: Roche Diagnostics GmbH, 68305 Mannheim (DE); F. Hoffmann-La Roche AG, 4070 Basel (CH)
(72) Inventor: NEWTON, Nicolas, Oakland, CA 94619 (US)
(74) Representative: Rauscher, Andreas
(86) International application number: PCT/EP2009/009053
(87) International publication number: WO 2010/072366

(56) References cited:
- EP-A1- 1 780 291
- EP-A1- 2 071 041
- WO-A2-2004/074447
- WO-A2-2005/090610

## Description

### FIELD OF THE INVENTION

The invention relates generally to a method for detecting a target nucleic acid in a sample. More specifically, the invention relates to a method for detecting a target nucleic acid in a sample using fluorescent probe pairs which include fluorescent reporter and quencher molecules which may be used in hybridization assays and in nucleic acid amplification reactions, especially polymerase chain reactions (PCR).

### BACKGROUND OF THE INVENTION

Methods using fluorescent probes for monitoring nucleic acid amplification reactions such as polymerase chain reactions (PCR) are known in the art.

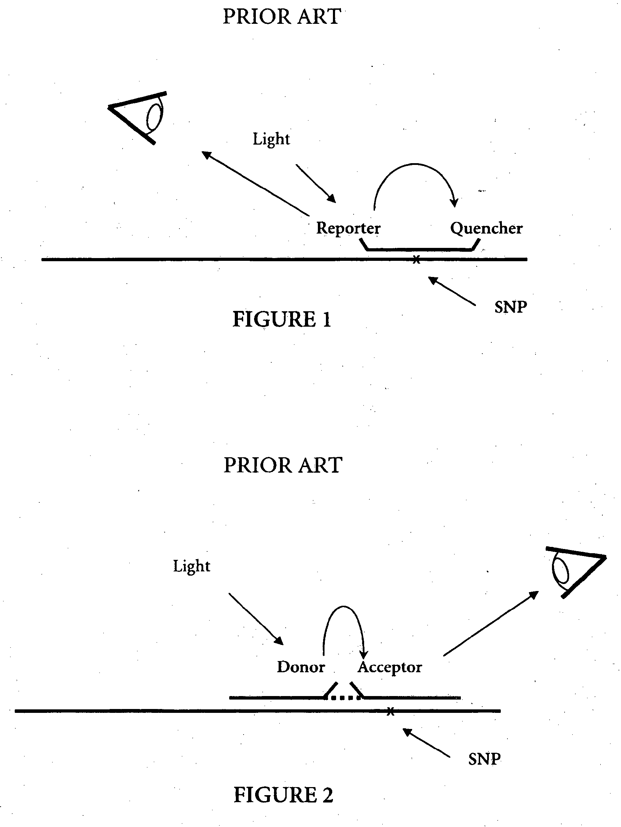

An example of fluorescent probes are fluorescent dual labeled probes as depicted in Figure 1. Such fluorescent dual labeled probes typically consist of an oligonucleotide labeled with two different dyes, namely a reporter and a quencher, able to hybridize to a specific target nucleic acid sequence. Reporters are molecules capable of emitting fluorescence when excited with light and quenchers are molecules capable of absorbing the fluorescence emitted by the reporter. In some cases, the reporter is located on the 5' end and the quencher is located on the 3' end of the oligonucleotide. With this type of fluorescent probe, fluorescence is emitted when the probe is hybridized to the target nucleic acid sequence or when it is cleaved during the PCR reaction. Monitoring of the PCR reaction is based on monitoring of the intensity of the fluorescence over time as the PCR reaction takes place. More specifically, when the probe is not hybridized to the target nucleic acid sequence it assumes a random coil conformation and the reporter is spatially close enough to the quencher that a Förster-type Resonance Energy Transfer (FRET) occurs from the reporter to the quencher. In this case, when the reporter is excited with light of the proper wavelength, the fluorescence emitted by the reporter is "quenched" by the quencher and a relatively lower fluorescence intensity is observed. When the probe is hybridized to the target nucleic acid sequence, the probe is no longer folded in on itself, and the distance between the reporter and the quencher increases. Consequently, the FRET is reduced, the fluorescence emitted by the reporter is less quenched by the quencher and a relatively higher fluorescence intensity is observed. If the probe is cleaved by the DNA polymerase during the elongation phase of a PCR, the reporter is severed from the probe and no FRET occurs between the reporter and the quencher. As a result, fluorescence intensity is also relatively higher. This process repeats in every cycle of the PCR reaction. A monitoring of the changes in the fluorescence intensity can hence be used to quantitatively monitor a PCR reaction.

There are some drawbacks associated with the use of fluorescent dual labeled probes. In particular, when a dual labeled probe is used in a PCR process with non-cleaving DNA polymerases, the magnitude of the fluorescent signal change is controlled only by the length and conformation of the DNA probe to which the dyes are attached. Since in the case of dual labeled probes, the dyes are attached to the same piece of DNA, the distance that they can move away from each other is limited. The applications of fluorescent dual labeled probes may be limited by this aspect.

By using a hybridization probe pair, where the fluorescent dyes are on different oligonucleotides, the dyes can move further away from each other, thus increasing the fluorescence change and resulting in greater signal dynamics. As schematically depicted in Figure 2, in fluorescent hybridization probe pairs, a first oligonucleotide is labeled with a fluorescent donor dye and a second oligonucleotide is labeled with a fluorescent acceptor dye. The first and the second oligonucleotides are designed to hybridize to a target nucleic acid sequence so that the donor and the acceptor are adjacent, or in close proximity, when both oligonucleotides are hybridized to the target nucleic acid sequence. When the donor dye is excited by light, it transfers its all or a portion of its energy to the acceptor by FRET. Light emitted by the acceptor can then be detected. A relatively higher fluorescence intensity indicates that the probes are hybridized to the target nucleic acid sequence. When the probes are cleaved by a DNA polymerase during the elongation phase of the PCR reaction, the donor and/or the acceptor are severed from the probes and a relatively lower fluorescence intensity is observed.

The drawback with fluorescent hybridization probe pairs is that a high fluorescent background of the fluorescent donor dye is observed when monitoring the fluorescence of the acceptor dye.

There is hence a need for an improved method for monitoring PCR reactions using fluorescent probes, wherein a high fluorescent background is eliminated and even greater signal dynamics are obtained.

### SUMMARY OF THE INVENTION

The invention provides a method for monitoring PCR reactions using fluorescent probes that fulfils this need.

In a first aspect, the invention relates to a method for detecting a target nucleic acid in a sample comprising:
- contacting said sample with a nucleic acid polymerase substantially lacking 5'-3' nuclease activity and a pair of a first and a second oligonucleotide probes under conditions wherein the first and second oligonucleotide probes selectively hybridize to said target nucleic acid, wherein the first oligonucleotide probe includes a fluorescent reporter and the second oligonucleotide probe includes a quencher so that the fluorescence of the fluorescent reporter is quenched by the quencher when the first and second oligonucleotide probes are hybridized to the target nucleic acid and the fluorescence of the fluorescent reporter is unquenched by the quencher when the first and second oligonucleotide are not hybridized to the target nucleic acid,
- exciting the fluorescent reporter with a light source,
- monitoring the fluorescence of the fluorescent reporter under conditions where the first and a second oligonucleotide probes are hybridized to the target nucleic acid, and comparing the fluorescence to that obtained under conditions where the first and second oligonucleotide probes are not hybridized to the target nucleic acid.

In another aspect, the invention relates to a kit for detecting a target nucleic acid in a sample comprising a pair of:
- a first oligonucleotide probe comprising a fluorescent reporter,
- a second oligonucleotide probe comprising a quencher effective to quench the fluorescence of the fluorescent reporter when the first and second oligonucleotide probes are hybridized to the target nucleic acid, and
- a nucleic acid polymerase substantially lacking 5'-3' nuclease activity.

In yet another aspect, the invention relates to a reaction mixture comprising:
- a target nucleic acid in a sample,
- a nucleic acid polymerase substantially lacking 5'-3' nuclease activity,
- a first oligonucleotide probe comprising a fluorescent reporter, and
- a second oligonucleotide probe including a quencher effective to quench the fluorescence of the fluorescent reporter when the first and second oligonucleotide probes are hybridized to the target nucleic acid.

### BRIEF DESCRIPTION OF THE DRAWINGS

Figure 1 is a schematic representation of a dual labeled probe according to the prior art.
Figure 2 is a schematic representation of an hybridized probe pair comprising a first oligonucleotide probe including a fluorescent donor and a second oligonucleotide probe including an acceptor according to prior art.
Figure 3 is a schematic representation of an hybridized probe pair comprising a first oligonucleotide probe including a fluorescent reporter and a second oligonucleotide probe including a quencher according to the method of the invention.
Figure 4 (a-b) shows fluorescent data represented by melting curves (4-a) and melting peaks (4-b) from a control fluorescent probe comprising a a fluorescent donor and an acceptor according to prior art.
Figure 5 (a-b) shows fluorescent data represented by melting curves (5-a) and melting peaks (5-b) from the excitation of a probe pair comprising a first oligonucleotide probe including a fluorescent reporter and a second oligonucleotide probe including a quencher according to the method of the invention.
Figure 6 shows an overlay of the fluorescent data of Figures 4 (b) and 5 (b).

### DEFINITIONS

To facilitate the understanding of this disclosure, the following definitions may be helpful.

As used herein, a "sample" refers to any substance containing or presumed to contain nucleic acid. This includes a sample of tissue or fluid isolated from an individual or individuals, including but not limited to, for example, skin, plasma, serum, spinal fluid, lymph fluid, synovial fluid, urine, tears, blood cells, organs, tumors, and also to samples of in vitro cell culture constituents (including but not limited to conditioned medium resulting from the growth of cells in cell culture medium, recombinant cells and cell components).

As used herein, the terms "nucleic acid", "polynucleotide" and "oligonucleotide" will be used interchangeably. These terms refer only to the primary structure of the molecule. Thus, these terms include double- and single-stranded DNA, as well as double- and single-stranded RNA. An oligonucleotide may be comprised of a sequence of approximately at least 6 nucleotides, for example at least about 10-12 nucleotides, or at least about 15-20 nucleotides corresponding to a region of the designated nucleotide sequence. "Corresponding" means identical to or complementary to the designated sequence. The oligonucleotide is not necessarily physically derived from any existing or natural sequence but may be generated in any manner, including chemical synthesis, DNA replication, reverse transcription or a combination thereof.

The term "primer" may refer to one or more than one primer and refers to an oligonucleotide, whether occurring naturally, as in a purified restriction digest, or produced synthetically, which is capable of acting as a point of initiation of synthesis along a complementary strand when placed under conditions in which synthesis of a primer extension product which is complementary to a nucleic acid strand is catalyzed.

The complement of a nucleic acid sequence as used herein refers to an oligonucleotide which, when aligned with the nucleic acid sequence such that the 5' end of one sequence is paired with the 3' end of the other, is in "antiparallel association." Complementarity need not be perfect; stable duplexes may contain mismatched base pairs or unmatched bases. Those skilled in the art of nucleic acid technology can determine duplex stability empirically considering a number of variables including, for example, the length of the oligonucleotide, base composition and sequence of the oligonucleotide, ionic strength, and incidence of mismatched base pairs.

As used herein, the term "target sequence", "target nucleic acid" or "target nucleic acid sequence" refers to a region of the oligonucleotide which is to be either amplified, detected or both. The target sequence resides between the two primer sequences used for amplification.

As used herein, the term "probe" refers to a labeled oligonucleotide which forms a duplex structure with a sequence in the target nucleic acid, due to complementarity of at least one sequence in the probe with a sequence in the target region. The probe, in certain embodiments, does not contain a sequence complementary to sequence(s) used to prime the polymerase chain reaction. The term "probes pair" refers to a pair of probes as described hereinabove.

The term "label" as used herein refers to any atom or molecule which can be used to provide a detectable (preferably quantifiable) signal, and which can be attached to a nucleic acid or protein. Labels may provide signals detectable by fluorescence, radioactivity, colorimetry, gravimetry, X-ray diffraction or absorption, magnetism, enzymatic activity, and the like.

The terms "hybridized" and "hybridization" refer to the base-pairing interaction of one oligonucleotide with another oligonucleotide (typically an antiparallel polynucleotide) that results in formation of a duplex or other higher-ordered structure, typically termed a hybridization complex. It is not a requirement that two polynucleotides have 100% complementarity over their full length to achieve hybridization. In some aspects, a hybridization complex can form from intermolecular interactions, or alternatively, can form from intramolecular interactions. Conversely, the expression "not hybridized" denotes a state wherein "hybridization" has not or not yet occurred.

As used herein, the terms "complementary" or "complementarity" are used in reference to antiparallel strands of polynucleotides related by the Watson-Crick and Hoogsteen-type base-pairing rules. For example, the sequence 5'-AGTTC-3' is complementary to the sequence 5'-GAACT-3'. The terms "completely complementary" or "100% complementary" and the like refer to complementary sequences that have perfect Watson-Crick pairing of bases between the antiparallel strands (no mismatches in the polynucleotide duplex). However, complementarity need not be perfect; stable duplexes, for example, may contain mismatched base pairs or unmatched bases. The terms "partial complementarity," "partially complementary," "incomplete complementarity" or "incompletely complementary" and the like refer to any alignment of bases between antiparallel polynucleotide strands that is less than 100% perfect (e.g., there exists at least one mismatch or unmatched base in the polynucleotide duplex). For example, the alignment of bases between the antiparallel polynucleotide strands can be at least 99%, 95%, 90%, 85%, 80%, 75%, 70%, 65%, 60%, 55%, or 50%, or any value between.

As used herein, the term "FRET" (fluorescent resonance energy transfer or Förster-type resonance energy transfer) and equivalent terms refers generally to a dynamic distance-dependent interaction between electron states of two dye molecules in which energy is transferred from a donor dye molecule to an acceptor dye molecule without emission of a photon from the donor molecule. The efficiency of FRET is dependent on the inverse of the intermolecular separation between the dyes, making it useful over distances comparable with the dimensions of biological macromolecules. Generally, FRET allows the imaging, kinetic analysis and/or quantitation of colocalizing molecules or conformational changes in a single molecule with spatial resolution beyond the limits of conventional optical microscopy. In general, FRET requires, (a) the donor and acceptor molecules must be in close proximity (typically, *e.g*., 10-100 A), (b) the absorption spectrum of the acceptor must overlap the fluorescence emission spectrum of the donor, and (c) the donor and acceptor transition dipole orientations must be approximately parallel.

In most FRET applications, the donor and acceptor dyes or reporter and quencher are different, in which case FRET can be detected by the appearance of sensitized fluorescence of the acceptor or by quenching of donor fluorescence. In some cases, the donor and acceptor or reporter and quencher are the same, and FRET can be detected by the resulting fluorescence depolarization. Use of a single donor/acceptor or reporter/quencher molecule in a FRET system is described, for example, in Published US Patent Application No. 2004/0096926, by Packard and Komoriya, published May 20, 2004, entitled "COMPOSITIONS FOR THE DETECTION OF ENZYME ACTIVITY IN BIOLOGICAL SAMPLES AND METHODS OF USE THEREOF".

FRET has become an important technique for investigating a variety of biological phenomena that are characterized by changes in molecular proximity. FRET techniques are now pervasive in many biological laboratories, and have been adapted for use in a variety of biological systems, including but not limited to, detection of nucleic acid hybridization, real-time PCR assays and SNP detection, structure and conformation of proteins, spatial distribution and assembly of protein complexes, reporter/ligand interactions, immunoassays, probing interactions of single molecules, structure and conformation of nucleic acids, primer-extension assays for detecting mutations, automated DNA sequencing, distribution and transport of lipids, membrane fusion assays (lipid-mixing assays of membrane fusion), membrane potential sensing, fluorogenic protease substrates, and indicators for cyclic AMP and zinc.

As used herein, the term "FRET reporter" refers typically to a moiety that produces a detectable emission of radiation, *e.g*., fluorescent or luminescent radiation, that can be transferred to a suitable FRET quencher in sufficient proximity. Generally, such molecules are dyes. The expression "FRET reporter" can be used interchangeably with "reporter" or "FRET label" or "FRET label moiety."

As used herein, the term "quencher" refers generally to a moiety that reduces and/or is capable of reducing the detectable emission of radiation, for example but not limited to, fluorescent or luminescent radiation, from a source that would otherwise have emitted this radiation. Generally, a quencher refers to any moiety that is capable of reducing light emission. The degree of quenching is not limited, *per se*, except that a quenching effect should minimally be detectable by whatever detection instrumentation is used. In some aspects, a quencher reduces the detectable radiation emitted by the source by at least 50%, alternatively by at least 80%, and alternatively and most preferably by at least 90%. In some embodiments, the quencher results in a reduction in the fluorescence emission from a reporter, and thus the reporter/quencher forms a FRET pair, and the quencher is termed a "FRET quencher" and the reporter is a "FRET reporter". It is not intended that that the term "quencher" be limited to FRET quenchers. For example, quenching can involve any type of energy transfer, including but not limited to, photoelectron transfer, proton coupled electron transfer, dimer formation between closely situated fluorophores, transient excited state interactions, collisional quenching, or formation of non-fluorescent ground state species. In some embodiments, a quencher refers to a molecule that is capable of reducing light emission. There is no requirement for a spectral overlap between the fluorophore and the quencher. As used herein, "quenching" includes any type of quenching, including dynamic (Förster-Dexter energy transfer, etc.), and static (ground state complex). Alternatively still, a quencher can dissipate the energy absorbed from a fluorescent dye in a form other than light, *e.g*., as heat. In some embodiments, some quenchers can re-emit the energy absorbed from a FRET reporter at a wavelength or using a signal type that is distinguishable from the FRET reporter emission, and at a wavelength or signal type that is characteristic for that quencher, and thus, in this respect, a quencher can also be a "label."

For general discussion on the use of fluorescence probe systems, see, for example, Principles of Fluorescence Spectroscopy, by Joseph R. Lakowicz, Plenum Publishing Corporation, 2nd edition (July 1, 1999) and Handbook of Fluorescent Probes and Research Chemicals, by Richard P. Haugland, published by Molecular Probes, 6th edition (1996).

A wide variety of dyes, fluors, quenchers, and fluorescent proteins, along with other reagents and detection/imaging instrumentation have been developed for use in FRET analysis and are widely commercially available. One of skill in the art recognizes appropriate FRET protocols, reagents and instrumentation to use for any particular analysis.

The term "monitoring" means monitoring the fluorescence emitted by the probes. This may also include detecting and measuring the intensity of the fluorescence, collecting and recording the fluorescence intensity data, for example on a computer readable medium of the types known in the art. Further steps include mathematical treatment of the data, analyses and interpretations thereof either with or without a computer.

The terms "nucleic acid polymerase substantially lacking the 5'-3' nuclease activity" or "5'-3'-nuclease-deficient enzyme", or for simplicity, "nuclease-deficient enzyme" refer to a polymerase that has 50% or less of the 5'-3' activity of native Taq DNA polymerase. The methods of measuring the 5'-3' nuclease activity and conditions for measurement have been described in U.S. Patent No. 5,466,591. Examples of polymerases lacking the 5'-3', nuclease activity include the Stoffel fragment of Taq DNA polymerase (U.S. Patent No. 5,466,591), mutants of *Thermus africanus* DNA polymerase (U.S. Patent No. 5,968,799), mutants of *Thermotoga maritima* DNA polymerase (U.S. Patent Nos. 5,624,833 and 5,420,029), mutants of *Thermus* species sps17 and *Thermus* species Z05 DNA polymerases (U.S. Patent Nos. 5,466,591 and 5,405,774). 5'-3' nuclease deficient enzymes may also be chimeras, i.e. chimeric proteins, composed of domains derived from more than one species and having mutations that eliminate the 5'-3' nuclease activity (e.g., as described in U.S. Patent Nos. 5,795,762 and 6,228,628).

As used herein, the term "Tₘ" is used in reference to the "melting temperature." The melting temperature is the temperature at which one half of a population of doublestranded polynucleotides or nucleobase oligomers (e.g., hybridization complexes), in homoduplexes or heteroduplexes, become dissociated into single strands. The prediction of a Tₘ of a duplex polynucleotide takes into account the base sequence as well as other factors including structural and sequence characteristics and nature of the oligomeric linkages. Methods for predicting and experimentally determining Tₘ are known in the art. For example, a Tₘ is traditionally determined by a melting curve, wherein a duplex nucleic acid molecule is heated in a controlled temperature program, and the state of association/dissociation of the two single strands in the duplex is monitored and plotted until reaching a temperature where the two strands are completely dissociated. The Tₘ is read from this melting curve. Alternatively, a Tₘ can be determined by an annealing curve, wherein a duplex nucleic acid molecule is heated to a temperature where the two strands are completely dissociated. The temperature is then lowered in a controlled temperature program, and the state of association/dissociation of the two single strands in the duplex is monitored and plotted until reaching a temperature where the two strands are completely annealed. The Tₘ is read from this annealing curve.

The practice of the present invention will employ, unless otherwise indicated, conventional techniques of chemistry, molecular biology, microbiology and recombinant DNA techniques, which are within the skill of the art. Such techniques are explained fully in the literature. See, e.g., Sambrook, Fritsch & Maniatis, Molecular Cloning; A Laboratory Manual, Second Edition (1989); Oligonucleotide Synthesis (M. J. Gait, ed., 1984); Nucleic Acid Hybridization (B. D. Hames & S. J. Higgins, eds., 1984); A Practical Guide to Molecular Cloning (B. Perbal, 1984); and a series, Methods in Enzymology (Academic Press, Inc.).

### DETAILED DESCRIPTION OF THE INVENTION

The invention provides a method for monitoring PCR reactions using fluorescent probes. In a first aspect, the invention relates to a method for detecting a target nucleic acid in a sample comprising: contacting said sample with a nucleic acid polymerase substantially lacking 5'-3' nuclease activity and a first and a second oligonucleotide probe, under conditions wherein said first and second oligonucleotide probes selectively hybridize to said target nucleic acid, wherein the first oligonucleotide probe includes a fluorescent reporter and the second oligonucleotide probe includes a quencher so that the fluorescence of the fluorescent reporter is quenched by the quencher when the first and second oligonucleotide probes are hybridized to the target nucleic acid and the fluorescence of the fluorescent reporter is unquenched by the quencher when the first and second oligonucleotide are not hybridized to the target nucleic acid; exciting the fluorescent reporter with a light source; monitoring the fluorescence of the fluorescent reporter under conditions where the first and second oligonucleotide probes are hybridized to the target nucleic acid; and comparing the fluorescence obtained under conditions where the first and second oligonucleotide probes are hybridized to the target nucleic acid to the fluorescence obtained when the probes are not hybridized to the target nucleic acid.

In the method of the invention the reporter dye emits fluorescence when excited with light, which fluorescence is directly monitored. The quencher attenuates the fluorescence of the reporter when it is spatially close enough to the reporter. This differs from methods of the prior art using fluorescent hybridization pairs with a donor and an acceptor, wherein, when excited by light, the donor transfers energy by FRET to the acceptor which in turn emits fluorescence. The acceptor then has its fluorescence monitored. By monitoring the fluorescence directly emitted by the reporter according to the method of the invention, the inventor discovered that the unwanted high fluorescence background of the donor in a donor-acceptor probe pair could be avoided. This is particularly beneficial in multiplex assays where many oligonucleotides probes bearing a donor are present. Also, the inventor discovered that more signal or greater signal dynamics were obtained from direct excitation as opposed to energy transfer.

Molecules commonly used in FRET as reporters or quenchers include, for example but not limited to, fluorescein dyes (e.g., FAM, JOE, and HEX), rhodamine dyes (e.g, R6G, TAMRA, ROX), and cyanine dyes (e.g, Cy3, Cy3.5, Cy5, Cy5.5, and Cy7). Other examples include DABCYL and EDANS. Whether a fluorescent dye acts as a reporter or a quencher is defined by its excitation and emission spectra, and by the fluorescent dye with which it is paired. For example, FAM is most efficiently excited by light with a wavelength of 488 nm, and emits light with a spectrum of 500 to 650 nm, and an emission maximum of 525 nm. FAM is a suitable reporter label for use with, *e.g*., TAMRA as a quencher, which has its excitation maximum at 514 nm. Examples of non-fluorescent or dark quenchers that dissipate energy absorbed from a fluorescent dye include the Black Hole Quenchers™ marketed by Biosearch Technologies, Inc. (Novato, CA, USA). The Black Hole Quenchers™ are structures comprising at least three radicals selected from substituted or unsubstituted aryl or heteroaryl compounds, or combinations thereof, wherein at least two of the residues are linked via an exocyclic diazo bond (see, *e.g*., International Publication No. WO 01/86001, entitled "DARK QUENCHERS FOR DONOR-ACCEPTOR ENERGY TRANSFER," published November 15, 2001 by Cook et al.). Other dark quenchers include Iowa Black querichers (*e.g.*, Iowa Black FQ^{™} and Iowa Black RQ^{™}) and Eclipse^{®} Dark Quenchers (Epoch Biosciences, Inc, Bothell, WA). Examples of quenchers are also provided in, *e.g.*, U.S. Patent No. 6,465,175, entitled "OLIGONUCLEOTIDE PROBES BEARING QUENCHABLE FLUORESCENT LABELS, AND METHODS OF USE THEREOF," which issued October 15, 2002 to Horn et al.

Fluorescent dyes include e.g., a rhodamine dye, e.g., R6G, R110, TAMRA, ROX, etc., see U.S. Pat. Nos. 5,366,860; 5,847,162; 5,936,087; 6,051,719; 6,191,278, a fluorescein dye e.g., JOE, VIC, TET, HEX, FAM, etc.; 6-carboxyfluorescein; 2',4',1,4,-tetrachloronuorescein; and 2',4',5',7', 1,4-hexachlorofluorescein; see U.S. Pat. Nos. 5,188,934; 6,008,379; 6,020,481, benzophenoxazines (U.S. Pat. No. 6,140,500), a halofluorescein dye, a cyanine dye (e.g., CY3, CY3.5, CY5, CY5.5, CY7, etc., see Published International Application No. WO 97/45539 by Kubista), a BODIPY® dye (e.g., FL, 530/550, TR, TMR, etc.), an ALEXA FLUOR® dye (e.g., 488, 532, 546, 568, 594, 555, 653, 647, 660, 680, etc.), a dichlororhodamine dye, an energy transfer dye (e.g., BIGDYE™ v 1 dyes, BIGDYE™ v 2 dyes, BIGDYE™ v 3 dyes, etc.), Lucifer dyes (e.g., Lucifer yellow, etc.), CASCADE BLUE®, Oregon Green, and the like. Additional examples of fluorescent dyes are provided in, e.g., Haugland, Molecular Probes Handbook of Fluorescent Probes and Research Products, Ninth Ed. (2003) and the updates thereto. Fluorescent dyes are generally readily available from various commercial suppliers including, e.g., Molecular Probes, Inc. (Eugene, OR), Amersham Biosciences Corp. (Piscataway, NJ), Applied Biosystems (Foster City, CA), etc.

FRET labeling techniques are commonly used in both real-time amplicon quantitation and for monitoring nucleic acid probe hybridization. In some embodiments, FRET label systems are used with the probes of the invention. It is not intended that the invention be limited to any particular FRET pair system. One of skill in the art recognizes the wide range of FRET labels that can be used with the probes of the invention. Fluorescent energy-transfer dye pairs of reporters and quenchers include, e.g., U.S. Pat. Nos. 5,863,727; 5,800,996; 5,945,526, as well as any other fluorescent label capable of generating a detectable signal.

Other labels that can be used as reporters and/or quenchers include, *e.g*., biotin, weakly fluorescent labels (Yin et al. (2003) Appl Environ Microbiol. 69(7):3938, Babendure et al. (2003) Anal. Biochem. 317(1):1, and Jankowiak et al. (2003) Chem Res Toxicol. 16(3):304), non-fluorescent labels, colorimetric labels, chemiluminescent labels (Wilson et al. (2003) Analyst. 128(5):480 and Roda et al. (2003) Luminescence 18(2):72), Raman labels, electrochemical labels, bioluminescent labels (Kitayama et al. (2003) Photochem Photobiol. 77(3):333, Arakawa et al. (2003) Anal. Biochem. 314(2):206, and Maeda (2003) J. Pharm. Biomed. Anal. 30(6):1725), and an alpha-methyl-PEG labeling reagent as described in, e.g., U.S. Patent Application Serial No. 10/719,257, filed on Nov. 21, 2003.

The preparation of the oligonucleotide probes can be made by methods known in the art. The person skilled in the art is capable of defining conditions where the oligonucleotide probes selectively hybridize to a target nucleic acid. Further, means and method for exciting and monitoring the fluorescent reporter are also known in the art.

In some embodiments of the invention, the amplification involves the polymerase chain reaction, i.e. repeated cycles of template denaturation, annealing (hybridization) of the oligonucleotide primer to the template, and extension of the primer by the nucleotide-incorporating biocatalyst. In some embodiments, the annealing and extension occur at the same temperature step.

In some embodiments, the amplification reaction involves a hot start protocol. In the context of allele-specific amplification, the selectivity of the allele-specific primers with respect to the mismatched target may be enhanced by the use of a hot start protocol. Many hot start protocols are known in the art, for example, the use of wax to separate the critical reagents from the rest of the reaction mixture (U.S. Patent No. 5,411,876), the use of a nucleic acid polymerase reversibly inactivated by an antibody (U.S. Patent No. 5,338,671), a nucleic acid polymerase reversibly inactivated by an oligonucleotide that is designed to specifically bind its active site (U.S. Patent No. 5,840,867) or the use of a nucleic acid polymerase with reversible chemical modifications, as described e.g. in U.S. Patent Nos. 5,677,152 and 5,773,528.

In some embodiments of the invention, the amplification assay is a real-time PCR assay. In a real-time PCR assay, the measure of amplification is the "cycles to threshold" or Ct value. An earlier Ct value reflect the rapid achievement of the threshold level and thus a more efficient amplification. The later Ct value may reflect inefficient or inhibited amplification. In the context of a real-time PCR assay, the difference in Ct values between the matched and the mismatched templates is a measure of the discrimination between the alleles or the selectivity of the assay.

Amplification of nucleic acid sequences, both RNA and DNA, is described in U.S. Pat. Nos. 4,683,195,4,683,202, and 4,965,188. The preferred method, polymerase chain reaction (PCR), typically is carried out using a thermostable DNA polymerase, which is able to withstand the temperatures used to denature the amplified product in each cycle. PCR is now well known in the art and has been described extensively in the scientific literature. See, for example, PCR Applications, ((1999) Innis et al., eds., Academic Press, San Diego), PCR Strategies, ((1995) Innis et al., eds., Academic Press, San Diego); PCR Protocols, ((1990) Innis et al., eds., Academic Press, San Diego), and PCR Technology, ((1989) Erlich, ed., Stockton Press, New York). A review of amplification methods is provided in Abramson and Myers, ((1993) Current Opinion in Biotechnology 4:41-47).

The method of the invention utilizes one or more nucleic acid polymerase(s) substantially lacking 5'-3' nuclease activity. Many of these polymerases, also known as 5'-3' nuclease-deficient enzymes, are known in the art. Some examples include G46E E678G CS5 polymerase, G46E E678G CS6 polymerase, TMA-25 polymerase, TMA-30 polymerase, delta-ZO5 polymerase, and 5'-3' nuclease-deficient mutants of Taq DNA polymerase known as the Stoffel fragment, described in U.S. Patent No. 5,466,591. If an enzyme that has 5'-3' nuclease activity is used, some fraction of the probe(s) are cleaved during PCR. This has two effects: the probes that have been cleaved can not participate in the post-PCR melt, resulting in reduced signal generation, and the cleaved fluorescent DNA fragments result in a higher and noisier fluorescent baseline. If a nuclease deficient enzyme is used, the probes are not cleaved, resulting in an assay with increased signal dynamics, and a less noisy baseline, which results in a more robust and sensitive assay.

In a certain embodiment, the method of the invention is applied to multiplex assays for detecting one or more species in a sample. In a typical multiplex assay, two or more distinct target nucleic acid sequences are detected using two or more probe pairs, each probe pair comprising a first and a second oligonucleotide probe, wherein each of the first oligonucleotide probe includes a different fluorophore and each of the second oligonucleotide probe includes a different quencher, capable of quenching the corresponding fluorophore.

Figure 1 is a schematic representation of a method according to the prior art using dual labeled probe comprising a reporter and a quencher on the same oligonucleotide. The dual labeled probe is hybridized to a target containing a Single Nucleotide Polymorphism (SNP). As already explained above, fluorescent dual labeled probes typically consist of an oligonucleotide labeled with two different dyes, namely a reporter and a quencher, able to hybridize to a specific target nucleic acid sequence. Reporters are molecules capable of emitting fluorescence when excited with light and quenchers are molecules capable of absorbing the fluorescence emitted by the reporter. With this type of fluorescent probe, fluorescence is emitted when the probe is hybridized to the target nucleic acid sequence or when it is cleaved during the PCR reaction. Monitoring of the PCR reaction is based on monitoring of the intensity of the fluorescence over time as the PCR reaction takes place. More specifically, when the probe is not hybridized to the target nucleic acid sequence, the probe assumes a random coil conformation and the reporter is spatially close enough to the quencher that a Förster-type Resonance Energy Transfer (FRET) occurs from the reporter to the quencher. In this case, when excited with light, the fluorescence emitted by the reporter is "quenched" by the quencher and a relatively lower fluorescence intensity is observed. When the probe is hybridized to the target nucleic acid sequence, the probe no longer has a random coil conformation and the distance between the reporter and the quencher is greater and the FRET is therefore reduced. Fluorescence emitted by the reporter is less quenched by the quencher and a relatively higher fluorescence intensity is observed. If the probe is cleaved by the DNA polymerase during the elongation phase of the PCR reaction, the reporter is severed from the probe and no FRET occurs between the reporter and the quencher. As a result, fluorescence intensity is also relatively higher. This process repeats in every cycle of the PCR reaction. A monitoring of the changes in the fluorescence intensity can hence be used to quantitatively monitor a PCR reaction.

Figure 2 is a schematic representation of a method according to the prior art using a labeled probe pair comprising two oligonucleotides, one bearing a donor and the other bearing an acceptor. The probe pair is hybridized to a target containing a Single Nucleotide Polymorphism (SNP). When the energy transfer hybridization probe pair is hybridized to the target, the donor is excited and transfers its energy to the acceptor, which produces a high fluorescence. Conversely, when the direct excitation hybridization probe pair is hybridized to the target, the fluorescence of the reporter is quenched by the quencher and fluorescence is relatively lower.

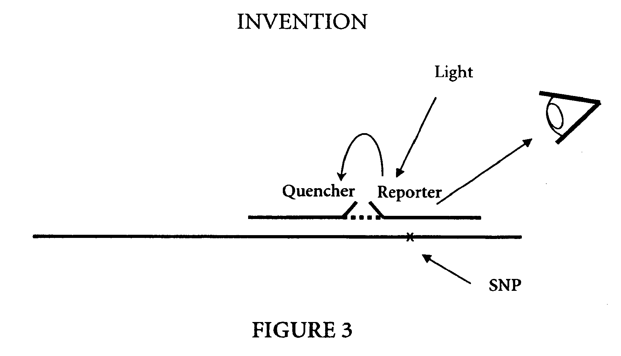

Figure 3 is a schematic representation of a method according to the invention using a labeled probe pair. The probe pair comprises a first oligonucleotide probe including a fluorescent reporter and a second oligonucleotide probe including a quencher. The probe pair is hybridized to a target containing a Single Nucleotide Polymorphism (SNP). When the probe pair is excited with light, the reporter is spatially close to the quencher and a transfer or energy from the excited reporter to the quencher occurs by FRET. The reporter's emission is relatively weak. When the probe pair is not hybridized the distance between the reporter and the quencher is more important than when the probe pair is hybridized to the target nucleic acid. As a result, the reporter's emission increases. The monitoring of the reporter's emission and its intensity changes can be used to perform analysis on the sample. For example, probe pairs can be designed to detect a certain target nucleic acid sequence. A monitoring of the fluorescence emitted by the reporter can indicate the presence of the target nucleic acid in the sample. This can lead to several applications in the diagnostic field, where the target nucleic acid can for example be the indication of a disease or condition.

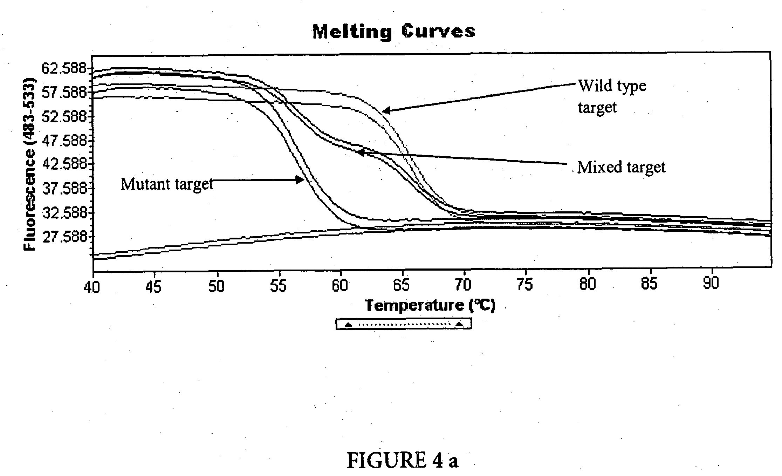

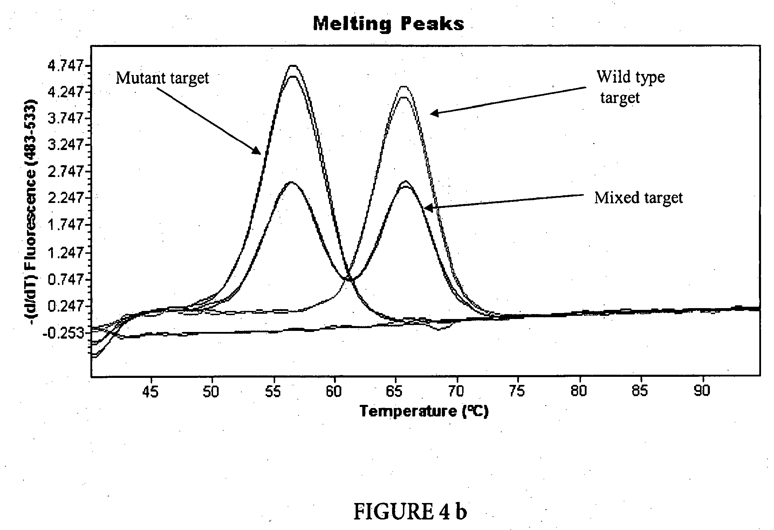

Figure 4 represents data from the type of prior art probe shown in Figure 1, where the reporter and quencher are on the same oligonucleotide. When the oligonucleotide is hybridized to the target, the reporter and quencher are maximally separated and the fluorescent signal is high. As the probe melts off the target as the temperature is raised, the probe adopts a random coil conformation, the reporter and quencher move closer together and the fluorescence goes down. This can be observed in the first panel (a) of Figure 4, which is the raw data of fluorescence against temperature in a post PCR melt assay for Factor V. This data is also known as "melting curve" data. The second panel (b) shows the negative first derivative of the raw data, or "melting peak" data, which gives a peak at the melting temperature (Tm) of the probe to the 3 targets in the experiment, in this case a wild type target which is perfectly matched to the probe and gives the highest Tm, a mutant target which has single base mismatch to the probe and gives a lower Tm, and a heterozygote target which contains both wild type and mutant targets in equal amounts and gives 2 peaks.

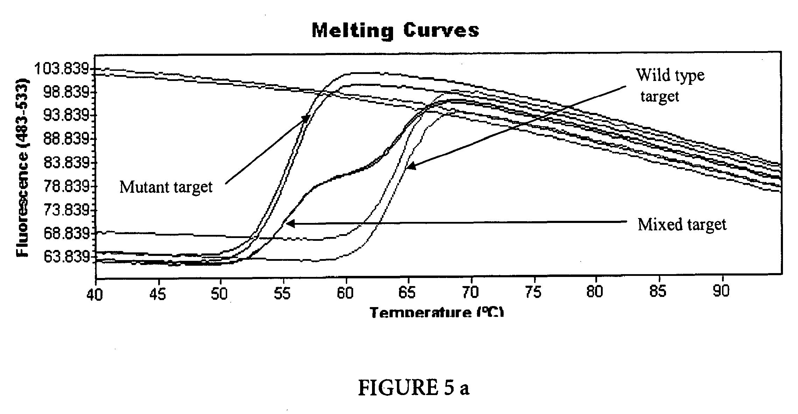

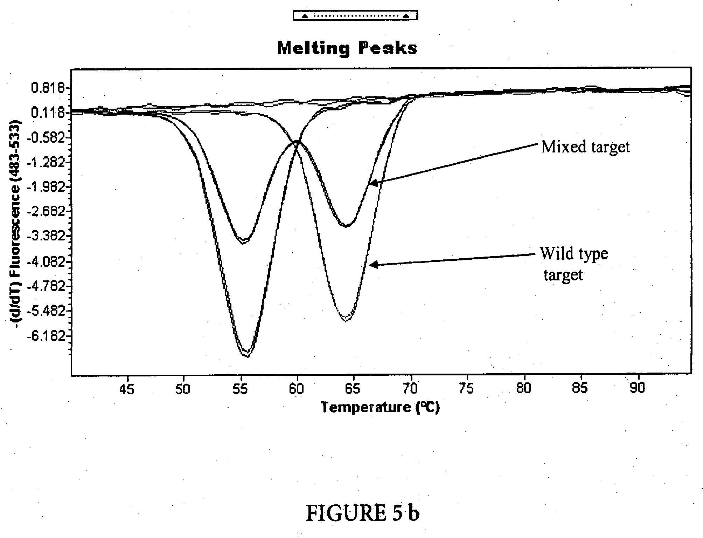

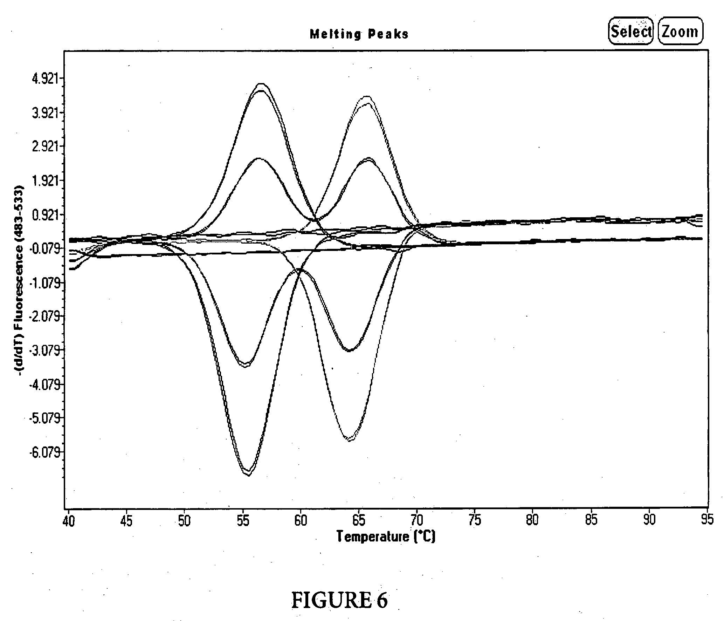

Figure 5 (a-b) shows data from the probe pair according to the invention shown in Figure 3. In this case, the reporter and quencher are on different oligonucleotides, and when the reporter is both excited and has its fluorescence monitored, the fluorescence change is in the opposite direction as the previous example. When the oligonucleotides are hybridized, the reporter and quencher are close together and fluorescence is low. When the oligonucleotides melt off the target, the dyes are maximally separated and the fluorescence goes up. This can be seen in the first panel (a) which shows the raw data of the post PCR melt of the same Factor V assay as in the previous example. The second panel (b) shows the negative first derivative of the raw data and shows negative peaks for the Tm values. Comparing the Y-axes for Figure 4 and 5, it can be seen that the magnitude of the signal for the probe pair (Figure 5) is significantly higher than that of the single probe (Figure 4). This can be seen more clearly if the negative derivative curves are plotted on the same graph (Figure 6).

Figure 6 shows the data from Figures 4 (b) and 5 (b) together. It can be seen that the magnitude of the signal from the hybridized probe pair (negative curves) is greater than from the dual labeled control probe (positive curves) by approximately 50%, which means that a greater signal dynamic is obtained.

As already explained above, the method of the invention overcomes many drawbacks of the detection methods of the prior art. Greater signal dynamics and therefore better accuracy are obtained.

The present invention also provides kits useful for employing the method of the invention. In a certain embodiment, the kit comprises a first oligonucleotide probe including a fluorescent reporter, a second oligonucleotide probe including a quencher so that the fluorescence of the fluorescent reporter is quenched by the quencher when the first and second oligonucleotide are hybridized to the target nucleic acid. Optionally, the kits can include paper or electronic instructions as well as a computer readable medium comprising a software for operating the monitoring of the fluorescence. The computer readable medium may be of a type that can be can be read or decoded by a computer or other instrument containing for example a microprocessor.

The present invention also provides reaction mixtures useful for employing the method of the invention. In a certain embodiment, the reaction mixture comprises a target nucleic acid in a sample, a first oligonucleotide probe including a fluorescent reporter, a second oligonucleotide probe including a quencher so that the fluorescence of the fluorescent reporter is quenched by the quencher when the first and second oligonucleotide are hybridized to the target nucleic acid. A typical reaction mixture will comprise the components used for amplification of nucleic acids.

The following example illustrates the present invention without limiting its scope to the embodiments described therein.

### Examples

In these examples, the method of the present invention was used to amplify a region of the human Factor V gene that includes the site of the Leiden mutation, cloned into a plasmid vector. In the current example, the asymmetric PCR sample master mix (100uL) consisted of: 5% glycerol; 50 mM Tricine, pH 8.3; 25 mM potassium acetate; 200 µM dATP, 200 µM dGTP, 200 µM dCTP, 400 µM dUTP; 0.7 µM upstream (excess) primer; 0.1µM downstream (limiting) primer; 0.4 µM each probe; 0.04U/µL uracil-N-glycosylase; 0.4 U-/µL ΔZO5 DNA polymerase; and 4 mM magnesium acetate.

The master mix was used to amplify Factor V wild type, mutant and mixed plasmid DNA targets. The excess primer was present at seven-fold excess over the limiting primer concentration to ensure an excess of single-stranded amplicon for the hybridization probe to bind to. The amplification and melting were performed on the Roche LightCycler 480.

The thermal cycling profile used for the example was: 50 °C for 5 minutes (UNG step); two cycles of 94°C for 15 seconds and then 59°C for 40 seconds; followed by 48 cycles of 91°C for 15 seconds and then 59°C for 40 seconds, with data collection during each 59°C step; and followed by a melting step with three data acquisitions per degree between 40°C and 95°C.

The sequence of the upstream primer was SEQ ID NO: 1, the sequence of the downstream primer was SEQ ID NO: 2, the sequence of dual labeled melting probe (control) was SEQ ID NO: 3, the sequence of the anchor probe was SEQ ID NO: 4, and the sequence of the acceptor probe was SEQ ID NO: 5. The sequences are shown in Table 1. The results of the experiment are shown on Figures 4-6.

Figure 4 shows melting curves (4a) and melting peaks (4b) obtained from a fluorescent probe comprising a donor and an acceptor. Figure 5 shows melting curves (5a) and melting peaks (5b) obtained from fluorescent probe pair comprising an oligonucleotide with a reporter and an oligonucleotide with a quencher. The distinct curves for the mutant, wild-type and mixed targets are indicated on both figures. Figure 6 shows an overlay of the fluorescent data of Figures 4 (b) and 5 (b).

**Table 1**

| *Primer and probe sequences* | | |
|---|---|---|
| SEQ ID NO 1 | Upstream primer | 5'-TGAACCCACAGAAAATGATGCCCE-3' |
| SEQ ID NO 2 | Downstream primer | 5'-GGAAATGCCCCATTATTTAGCCAGGE-3' |
| SEQ ID NO 3 | Melting probe | 5'-FCTGTATTCCTCGCCTGTCCAGQP-3' |
| SEQ ID NO 4 | Anchor probe | 5'-GAAATTCTCAGAATTTCTG-AAAGGTTACTTCAAGGACAAQP-3' |
| SEQ ID NO 5 | Acceptor probe | 5'-FCTGTATTCCTCGCCTGTCCAGP-3' |

| | | |
|---|---|---|
| E = t-butyl benzyl dA F = cx-FAM Q = BHQ2 P= 3'- phosphate | | |

## Claims

1. A method for detecting a target nucleic acid in a sample comprising:
a) amplifying said target nucleic acid in a reaction as PCR,
b) contacting said sample with a nucleic acid polymerase substantially lacking 5'-3' nuclease activity and a first and a second oligonucleotide probe, under conditions wherein said first and second oligonucleotide probes selectively hybridize to said target nucleic acid, wherein the first oligonucleotide probe includes a fluorescent reporter and the second oligonucleotide probe includes a quencher so that the fluorescence of the fluorescent reporter is quenched by the quencher when the first and second oligonucleotide probes are hybridized to the target nucleic acid and the fluorescence of the fluorescent reporter is unquenched by the quencher when the first and second oligonucleotide are not hybridized to the target nucleic acid,
c) exciting the fluorescent reporter with a light source,
d) monitoring the fluorescence of the fluorescent reporter in a post-PCR melt assay.

2. The method according to claim 1, wherein at least two pairs of oligonucleotide probes are used in a multiplex assay.

3. The method according to claim 1, wherein the reporter is selected from the group consisting of a fluorescein dye, a rhodamine dye, a cyanine dye, FAM, JOE, R6G, TAMRA, ROX, DABCYL, Cy3, Cy3.5, Cy5, Cy5.5, Cy7, and EDANS.

4. The method according to claim 1, wherein the quencher is selected from the group consisting of a fluorescein dye, a rhodamine dye, a cyanine dye, FAM, JOE, R6G, TAMRA, ROX, DABCYL, Cy3, Cy3.5, Cy5, Cy5.5, Cy7, and EDANS.

5. The method according to claim 1, wherein the quencher is a dark quencher.

6. The method according to claim 1, wherein the nucleic acid polymerase substantially lacking the 5'-3' nuclease activity is derived from one or more species selected from a group consisting of *Thermus aquaticus, Thermus* species *sps17, Thermus* species Z05, *Thermotoga maritima* and *Thermus africanus.*

7. The method according to claim 1, wherein detecting a characteristic increase in fluorescence measured in step d) involves plotting the first derivative of the fluorescence over temperature using the measurement in step d).

8. The method according to claim 7, wherein detecting a characteristic increase in fluorescence measured in step d) involves detecting a characteristic peak in the plot of the first derivative of the fluorescence over the temperature using the measurement in step d).

## Patentansprüche

1. Verfahren zum Nachweisen einer Zielnukleinsäure in einer Probe, umfassend:
a) Amplifizieren der Zielnukleinsäure in einer Reaktion wie PCR,
b) Inkontaktbringen der Probe mit einer Nukleinsäure-Polymerase, der eine 5'-3'-Nuklease-Aktivität weitgehend fehlt, und einer ersten und einer zweiten Oligonukleotidsonde unter Bedingungen, worin die erste und zweite Oligonukleotidsonde selektiv an die Zielnukleinsäure hybridisieren, wobei die erste Oligonukleotidsonde einen Fluoreszenz-Reporter und die zweite Oligonukleotidsonde einen Fluoreszenz-Quencher enthält, so dass die Fluoreszenz des Fluoreszenz-Reporters vom Quencher gelöscht ist, wenn die erste und zweite Oligonukleotidsonde an die Zielnukleinsäure hybridisiert sind, und die Fluoreszenz des Fluoreszenz-Reporters vom Quencher ungelöscht ist, wenn das erste und zweite Oligonukleotid nicht an die Zielnukleinsäure hybridisiert sind,
c) Anregen des Fluoreszenz-Reporters mit einer Lichtquelle,
d) Überwachen der Fluoreszenz des Fluoreszenz-Reporters in einem Post-PCR-Schmelztest.

2. Verfahren nach Anspruch 1, wobei wenigstens zwei Paar Oligonukleotidsonden in einem Multiplex-Testverfahren verwendet werden.

3. Verfahren nach Anspruch 1, wobei der Reporter aus der aus einem Fluorescein-Farbstoff, einem Rhodamin-Farbstoff, einem Cyanin-Farbstoff, FAM, JOE, R6G, TAMRA, ROX, DABCYL, Cy3, Cy3.5, Cy5, Cy5.5, Cy7 und EDANS bestehenden Gruppe ausgewählt ist.

4. Verfahren nach Anspruch 1, wobei der Quencher aus der aus einem Fluorescein-Farbstoff, einem Rhodamin-Farbstoff, einem Cyanin-Farbstoff, FAM, JOE, R6G, TAMRA, ROX, DABCYL, Cy3, Cy3.5, Cy5, Cy5.5, Cy7 und EDANS bestehenden Gruppe ausgewählt ist.

5. Verfahren nach Anspruch 1, wobei es sich bei dem Quencher um einen Dark Quencher handelt.

6. Verfahren nach Anspruch 1, wobei die Nukleinsäure-Polymerase, der eine 5'-3'-Nuklease-Aktivität weitgehend fehlt, aus einer oder mehreren aus einer aus *Thermus aquaticus, Thermus-Spezies sps17, Thermus-Spezies* Z05, *Thermotoga maritima* und *Thermus africanus* bestehenden Gruppe ausgewählten Spezies gewonnen wird.

7. Verfahren nach Anspruch 1, wobei Nachweisen eines charakteristischen Anstiegs der in Schritt d) gemessenen Fluoreszenz das graphische Auftragen der ersten Ableitung der Fluoreszenz gegen die Temperatur unter Verwendung der Messung in Schritt d) beinhaltet.

8. Verfahren nach Anspruch 1, wobei Nachweisen eines charakteristischen Anstiegs der in Schritt d) gemessenen Fluoreszenz Nachweisen eines charakteristischen Maximums im Graph der ersten Ableitung der Fluoreszenz gegen die Temperatur unter Verwendung der Messung in Schritt d) beinhaltet.

## Revendications

1. Procédé pour détecter un acide nucléique cible dans un échantillon, consistant à :
a) amplifier ledit acide nucléique cible dans une réaction telle qu'une PCR,
b) mettre en contact ledit échantillon avec une acide nucléique polymérase qui est dénuée substantiellement d'une activité de nucléase 5'-3' et une première et une deuxième sonde oligonucléotidique, dans des conditions dans lesquelles ladite première et ladite deuxième sonde oligonucléotidique s'hybrident sélectivement audit acide nucléique cible, la première sonde oligonucléotidique comprenant un rapporteur fluorescent et la deuxième sonde oligonucléotidique comprenant un extincteur de manière telle que la fluorescence du rapporteur fluorescent est éteinte par l'extincteur lorsque la première et la deuxième sonde oligonucléotidique sont hybridées à l'acide nucléique cible et la fluorescence du rapporteur fluorescent n'est pas éteinte par l'extincteur lorsque la première et la deuxième sonde oligonucléotidique ne sont pas hybridées audit acide nucléique cible,
c) exciter le rapporteur fluorescent par une source lumineuse,
d) suivre la fluorescence du rapporteur fluorescent dans un essai de fusion post-PCR.

2. Procédé selon la revendication 1, au moins deux paires de sondes oligonucléotidiques étant utilisées dans un essai multiplexe.

3. Procédé selon la revendication 1, le rapporteur étant choisi dans le groupe constitué par un colorant fluorescent, un colorant de type rhodamine, un colorant de type cyanine, FAM, JOE, R6G, TAMRA, ROX, DABCYL, Cy3, Cy3.5, Cy5, Cy5.5, Cy7 et EDANS.

4. Procédé selon la revendication 1, l'extincteur étant choisi dans le groupe constitué par un colorant fluorescent, un colorant de type rhodamine, un colorant de type cyanine, FAM, JOE, R6G, TAMRA, ROX, DABCYL, Cy3, Cy3.5, Cy5, Cy5.5, Cy7 et EDANS.

5. Procédé selon la revendication 1, l'extincteur étant un extincteur non phosphorescent.

6. Procédé selon la revendication 1, l'acide nucléique polymérase qui est dénuée substantiellement d'activité de nucléase 5'-3' étant dérivée d'une ou de plusieurs espèces choisies dans le groupe constitué par Thermus aquaticus, l'espèce Thermus sps17, l'espèce Thermus Z05, Thermotoga maritima et Thermus africanus.

7. Procédé selon la revendication 1, la détection d'une augmentation caractéristique de la fluorescence mesurée dans l'étape d) impliquant la mise en graphique de la première dérivée de la fluorescence par rapport à la température à l'aide de la mesure dans l'étape d).

8. Procédé selon la revendication 7, la détection d'une augmentation caractéristique de la fluorescence mesurée dans l'étape d) impliquant la détection d'un pic caractéristique dans le graphique de la première dérivée de la fluorescence par rapport à la température à l'aide de la mesure dans l'étape d).


### SEQUENCE LISTING

<110> F. Hoffmann-La Roche AG Roche Diagnostics GmbH
<120> METHOD FOR DETECTING A TARGET NUCLEIC ACID IN A SAMPLE
<130> 25746 WO
<140> not known
   <141> not known
<150> 12/341,130
   <151> 22.12.2008
<160> 5
<170> PatentIn version 3.5
<210> 1
   <211> 24
   <212> DNA
   <213> Artificial Sequence
<220>
   <223> Description of Artificial Sequence: Synthetic primer
<220>
   <221> modified_base
   <222> (24)..(24)
   <223> t-butyl benzyl dA
<400> 1
   tgaacccaca gaaaatgatg ccca 24
<210> 2
   <211> 26
   <212> DNA
   <213> Artificial Sequence
<220>
   <223> Description of Artificial Sequence: Synthetic primer
<220>
   <221> modified_base
   <222> (26)..(26)
   <223> t-butyl benzyl dA
<400> 2
   ggaaatgccc cattatttag ccagga 26
<210> 3
   <211> 21
   <212> DNA
   <213> Artificial Sequence
<220>
   <223> Description of Artificial Sequence: Synthetic probe
<400> 3
   ctgtattcct cgcctgtcca g 21
<210> 4
   <211> 39
   <212> DNA
   <213> Artificial Sequence
<220>
   <223> Description of Artificial Sequence: Synthetic probe
<400> 4
   gaaattctca gaatttctga aaggttactt caaggacaa 39
<210> 5
   <211> 21
   <212> DNA
   <213> Artificial Sequence
<220>
   <223> Description of Artificial Sequence: Synthetic probe
<400> 5
   ctgtattcct cgcctgtcca g 21